# EUROPÄISCHE PATENTSCHRIFT

(11) **EP 1 583 572 B1**
(45) Veröffentlichungstag und Bekanntmachung des Hinweises auf die Patenterteilung: **02.05.2007**
(21) Anmeldenummer: 04702689.3
(22) Anmeldetag: 16.01.2004
(51) Int. Cl.: A61M 5/158, A61L 29/14, A61L 31/14

(54) **BIEGSAME EINSTECHNADEL**
FLEXIBLE INJECTION NEEDLE
AIGUILLE A PIQUER SOUPLE

(30) Priorität: 17.01.2003 CH 73032003; 13.02.2003 DE 10306013
(43) Veröffentlichungstag der Anmeldung: 12.10.2005
(73) Patentinhaber: Disetronic Licensing AG, 3401 Burgdorf (CH)
(72) Erfinder: BARKHAHN, Susanne, CH-3007 Bern (CH); REINMANN, Andreas, CH-4542 Luterbach (CH)
(74) Vertreter: Schalch, Rainer
(86) Internationale Anmeldenummer: PCT/EP2004/000309
(87) Internationale Veröffentlichungsnummer: WO 2004/064898

(56) Entgegenhaltungen:
- EP-A- 0 529 675
- WO-A-02/28458
- US-A- 2 828 744
- US-A- 4 976 704
- US-A- 5 624 727
- US-A- 6 126 633

## Beschreibung

Die Erfindung betrifft eine Einstechnadel zum Einstechen in ein Körpergewebe, vorzugsweise in oder durch die menschliche Haut, die in eingeführtem Zustand biegsam ist ein zu verabreichendes Fluid in das Gewebe leitet. Die Einstechnadel bildet ein inneres Lumen zum Durchleiten des Fluids, d. h. sie bildet eine Einstechkanüle.

Bei einer Vielzahl therapeutischer oder diagnostischer Anwendungen ist es erforderlich, eine Einstechnadel über einen länger andauernden Zeitraum innerhalb eines Körpergewebes vorzusehen, um z.B. eine wiederholte oder lang andauernde Verabreichung therapeutischer oder diagnostischer Fluide ermöglichen zu können. Bei der Behandlung von Diabeteskranken wird z.B. Insulin in regelmäßigen Abständen durch eine Einstechnadel verabreicht, die der Patient über mehrere Tage in sein Körpergewebe eingebracht mit sich trägt.

Es ist z.B. aus der US 4,562,751 bekannt, hierfür eine Einstechnadel aus Stahl zu verwenden. Eine Stahlnadel weist zwar einen einfachen Aufbau auf, birgt jedoch verschiedene Nachteile. Die scharfe Nadelspitze der steifen Einstechnadel kann zu einer ständigen Irritation des umliegenden Gewebes fuhren, da sie sich nicht den Bewegungen des Gewebes anpassen kann. Eine solche steife eingebrachte Einstechnadel ist für den Patienten unangenehm und sogar schmerzhaft. Ferner besteht ein erhebliches Risiko von Nadelstichverletzungen, des die Nadelspitze umgebenden Gewebes sowie beim Entfernen der Einstechnadel aus dem Gewebe.

US 5,624,727 offenbart einen Festkörper, welcher auf Verlangen und aufgrund eines äusseren Impulses seine Steifheit reversibel verändern kann. Diese reversible Steifheistveränderung wird erreicht durch eine Umverteilung eines flüssigen Mediums, welche die Interaktion zwischen einer Vielzahl von festen Körpern oder Teilen im Festkörper beeinflusst. Solche Festkörper können z.B. in chirurgischen Geräten und Implantaten Anwendung finden.

Eine solche Softkanüle kann den Bewegungen des Gewebes problemlos folgen, so dass keine oder nur eine geringe Irritation des umliegenden Gewebes entsteht. Zum Einbringen der Softkanüle ist jedoch weiterhin eine steife Einstechnadel, wie eine Stahlnadel notwendig, die nach dem Einbringen der beschriebenen Softkanüle entfernt werden muss. Das Risiko einer Nadelstichverletzung ist somit auch bei der Softkanüle gegeben. Außerdem muss die Öffnung, durch welche die Stahlnadel entfernt wurde, für einen ordnungsgemäßen Einsatz der Einstechnadel abgedichtet werden. Dieser Vorgang ist umständlich und das Risiko einer undichten Stelle beim Einsatz der Kanüle steigt.

Es ist eine Aufgabe der vorliegenden Erfindung, die Handhabung und den Aufbau einer Einstechnadel zum Einstechen in ein Körpergewebe zu vereinfachen, die zum Einstechen notwendigen Einzelteile zu reduzieren, das Tragen der Einstechnadel angenehm zu gestalten und das Risiko von Stichverletzungen bei der Handhabung der Einstechnadel weiter zu vermindern.

Die Aufgabe wird durch eine Einstechnadel nach dem Patentanspruch 1 gelöst. Vorteilhafte Ausgestaltungen gehen aus den Unteransprüchen hervor.

Demnach geht die Erfindung von einer Einstechnadel zum Einstechen in ein Körpergewebe aus, die in eingeführtem Zustand biegsam ist und zum Einleiten eines Fluids vorzugsweise ein inneres Lumen aufweist. Vor dem Einführen in das Körpergewebe weist die Einstechnadel eine ausreichende Steifigkeit auf, um die Oberfläche des Körpergewebes zu durchdringen und weiter in das Gewebe eingeführt werden zu können. Erfindungsgemäß besteht die Einstechnadel wenigstens abschnittsweise aus einem Material, das in einem ersten Zustand biegesteif und gegebenenfalls auch im Hinblick auf andere Belastungen fest und in einem zweiten Zustand biegeweich, vorzugsweise elastisch, ist. Der erste Zustand ist dadurch charakterisiert, dass die Einstechnadel eine ausreichende Steifigkeit und zumindest an einer Spitze eine ausreichende Härte aufweist, um in das Körpergewebe eingestochen werden zu können. Der zweite Zustand ist dadurch charakterisiert, dass das verwendete Material z.B. in verschiedene Richtungen radial zu einer Längsachse der Einstechnadel biegbar ist oder komprimierbar und wieder expandierbar ist, aber zum Leiten des Fluids in das Gewebe ein ausreichendes inneres Lumen oder/und gegebenenfalls äußeres Lumen bildet.

Die biegeveränderliche Einstechnadel besteht in bevorzugten Ausführungen über ihre gesamte Länge oder zumindest abschnittsweise aus einem Material, das in dem ersten Zustand der Einstechnadel einen E-Modul von über 1000 MPa, vorzugsweise von 2000 MPa oder mehr aufweist. Das Material sollte sogar einen E-Modul von wenigstens 3000 MPa aufweisen. Durch das Einstechen oder vorzugsweise nach einer gewissen Zeit nach dem Einstechen, die nicht länger als 20-30 Minuten sein sollte, ändert sich der E-Modul des Materials und sollte dann nicht mehr als 1000 MPa betragen. Vorzugsweise ist sein E-Modul im zweiten Zustand kleiner als 800 MPa. Ein besonders bevorzugtes Material hat im ersten Zustand einen E-Modul von 3000 MPa oder darüber und im zweiten Zustand einen E-Modul von 700 MPa ± 100 MPa.

Das im E-Modul veränderliche Material kann ein homogenes Material oder ein Verbundmaterial sein. In bevorzugten Ausführungen kommt ein Formgedächtnismaterial, vorzugsweise ein Polymer oder ein Gel, beispielsweise ein Polymergel, oder auch eine Kombination mehrerer Formgedächtnismaterialien zum Einsatz.

Das im E-Modul veränderliche Material kann als Verbundmaterial insbesondere aus einem, eine Stützmatrix bildenden Material, vorzugsweise ein Kunststoffmaterial, und in der Stützmatrix eingebettetem Füllstoff gebildet sein. Der Füllstoff sollte aus einem harten und steifen Material mit einem E-Modul von vorzugsweise wenigstens 5000 MPa gebildet sein, das vorzugsweise in kleinen Partikeln vorliegt. Als Füllstoffmaterial kommen insbesondere Keramikmaterialien, aber auch Glas, Metall und sogar Polymerwerkstoffe in Frage. Der Füllstoff sollte einen Volumenanteil von wenigstens 5 % und höchstens 95 %, vorzugsweise höchstens 80 %, am Verbundmaterial haben. Statt kompakter Partikel kann der Füllstoff auch ein Fasermaterial sein, dessen Fasern vorzugsweise wesentlich kürzer als die Einstechnadel sind. Das Füllmaterial kann in der Stützmatrix über die Länge der Einstechnadel gleichmäßig verteilt sein, so dass die mittels des Füllmaterials erhaltende Versteifung im ersten Zustand der Einstechnadel über deren gesamte Länge gleich groß ist. Alternativ kann das Füllmaterial jedoch auch abschnittsweise unterschiedlich verteilt sein, um die Steifigkeit der Einstechnadel gezielt zu beeinflussen. So kann die Konzentration des Füllmaterials insbesondere an der Spitze der Einstechnadel größer als im Schaftbereich sein. In solch einer Ausführung beträgt die Konzentration, d. h. der Volumenanteil, des Füllmaterials an der Spitze zwischen 50 und 80 % und im Schaftbereich weniger als 50 %, vorzugsweise zwischen 5 und 20 % oder noch bevorzugter zwischen 10 und 20 %. Der Übergang zwischen einem Abschnitt mit einem hohen Füllmaterialanteil und einem Abschnitt mit einem demgegenüber niedrigeren Füllmaterialanteil ist vorzugsweise stetig, kann jedoch auch abrupt, d. h. unstetig, gestaltet sein. Eine Variation des Füllmaterialanteils, beispielsweise von der Spitze der Einstechnadel bis zu deren proximalen Ende abnehmend, kann beispielsweise durch Extrusion der Einstechnadel erzielt werden, indem dem Extruder das Füllmaterial in einem dem Volumenanteil in der Einstechnadel entsprechenden Volumenstrom zugeführt und im Extruder dem Material der Stützmatrix zugemischt wird. Das Stützmatrixmaterial ist vorzugsweise ein Formgedächtnismaterial, dessen E-Modul sich im eingestochenen Zustand oder bereits bei dem Einstechen selbst verringert. Das Material der Stützmatrix kann in Bezug auf den E-Modul die vorstehend beschriebenen Eigenschaften aufweisen. Durch die Einbettung des Füllmaterials kann der E-Modul des Stützmatrixmaterials außerhalb des Gewebes, d. h. vor dem Einstechen, allerdings auch geringer sein als bei einem E-Modul veränderlichen Material ohne eingebrachtes Füllmaterial.

Als im E-Modul veränderliche Materialien kommen insbesondere Kunststoffmaterialien in Frage, vorzugsweise thermoplastische Polymere. Die erfindungsgemäße Änderung der Materialeigenschaft, die vorzugsweise durch die Änderung des E-Moduls des betreffendes Materials ausgedrückt wird, beruht vorteilhafterweise auf dem Übergang in den amorphen Zustand. Der Übergang kann insbesondere durch die Änderung der Temperatur oder des pH-Werts oder auf einer chemischen Reaktion des Materials mit einem Medium der Umgebung oder auf einer Kombination von mehreren dieser Faktoren beruhen. Ein bevorzugtes Material ist ein thermoplastisches Polymer mit einer Glasübergangstemperatur von wenigstens 30 °C, vorzugsweise wenigstens 32 °C, und höchstens der Körpertemperatur, d. h. höchstens etwa 37 °C, vorzugsweise höchstens 36 °C. Anstatt ein Material mit einer Glastemperatur im gewünschten Bereich zu verwenden oder in Kombination mit solch einem Material, kann ein Material mit einer Schmelztemperatur bei oder unterhalb der Körpertemperatur, aber oberhalb der Umgebungstemperatur verwendet werden. Dementsprechend kann verallgemeinernd von einer Schalttemperatur gesprochen werden, die insbesondere die Glastemperatur oder die Schmelztemperatur oder grundsätzlich auch eine Temperatur sein kann, bei der ein anderer Übergang des Materials stattfindet, wenn nur die Nachgiebigkeit sich ändert, wenn die Nadel sich bis wenigstens auf die Schalttemperatur erwärmt hat. Die Schalttemperatur sollte wenigstens 30 °C betragen, damit die Nadel unter den üblichen Umgebungstemperaturen den ersten Zustand einnimmt.

Obgleich Kunststoffe bevorzugte Nadelmaterialien sind, kann die Einstechnadel grundsätzlich auch aus einem anderen Material bestehen, das körperverträglich ist und den Zustandswechsel durchläuft.

Anstatt die Änderung der Biegesteifigkeit der Einstechnadel durch eine Änderung des E-Moduls herbeizuführen, kann die Biegesteifigkeit auch durch eine Änderung des hierfür maßgeblichen Trägheitsmoments der Einstechnadel, d. h. durch Formänderung der Einstechnadel oder wenigstens eines Abschnitts der Einstechnadel herbeigeführt werden. Schließlich kann die Biegesteifigkeit auch durch eine Kombination einer Variation des E-Moduls und des Trägheitsmoments verändert werden.

Bevorzugt wird es, wenn die Spitze der Einstechnadel im zweiten Zustand weich ist, d. h. aus dem im E-Modul veränderlichen Material besteht. Grundsätzlich ist es jedoch auch möglich, die Spitze als dauerhaft fest zu bilden. Sie sollte dann jedoch möglichst kurz sein, zumindest so kurz, dass ein im eingestochenen Zustand biegeweicher Abschnitt der Einstechnadel sich an die in diesem Fall auch im eingestochenen Zustand feste Spitze anschließt, um ein schmerzminderndes Nachgeben, vorzugsweise Verbiegen, der Einstechnadel im eingestochenen Zustand zu gewährleisten.

In einer Ausführungsform ist die erfindungsgemäße Einstechnadel vollständig aus Formgedächtnismaterial hergestellt. Bevorzugt ist das Formgedächtnismaterial zur Ausbildung der Einstechnadel schlauchartig ausgebildet, sodass sich ein Innenraum als inneres Lumen zum Durchleiten des Fluids ausbilden kann. Im ersten Zustand weist der Schlauch aus Formgedächtnismaterial eine Form auf, in der am Umfang des Schlauches, bzw. der Einstechnadel, wenigstens eine spitze Kante ausgebildet ist, das heißt, die Kante weist einen spitzen Winkel auf, sodass sie als eine Art Schneidkante zum Einführen der Einstechnadel dienen kann. In diesem ersten Zustand muss kein Hohlraum im Inneren des Schlauches aus Formgedächtnismaterial ausgebildet sein. Vorzugsweise ist ein Hohlraum bzw. Lumen im ersten Zustand tatsächlich nicht gebildet. Die Innenflächen der Wandung des Schlauches liegen aneinander an. Weiter wird in diesem ersten Zustand an einem Ende der Einstechnadel, das zum Einstechen in das Körpergewebe dient, eine solide, vorzugsweise geschlossene, Spitze ausgebildet. Die Spitze der Einstechnadel weist dann keine Öffnung auf, sondern besteht aus einem massiven Bereich des Formgedächtnismaterials. Grundsätzlich kann die Spitze aber auch offen sein.

Schlauchartig verhält sich die Einstechnadel in der bevorzugten Ausführung als Einstechkanüle vorzugsweise im zweiten Zustand, während ihre Eigenschaften bezüglich des Einstechens selbst denen eines Rohres entsprechen, die Einstechnadel in der bevorzugten Ausführung als Einstechkanüle somit im ersten Zustand rohrartig ist. Das veränderliche Material kann jedoch auch im ersten Zustand schlauchartig sein, wenn es für den Erhalt der erforderlichen Steifigkeit zum Einstechen versteift ist. Wenn im Folgenden die Begriffe "Schlauch" und "schlauchartig" verwendet werden, so soll damit die Einstechnadel in der bevorzugten Ausführung als Einstechkanüle im zweiten Zustand beschrieben werden, wobei solch eine Einstechnadel im ersten Zustand rohrartig sein kann, aber nicht muss.

Im steifen ersten Zustand kann die Einstechnadel auf einfache Weise ohne weitere Hilfsmittel mittels ihrer Spitze in die Gewebeoberfläche eingestochen und mittels einer in Längsrichtung der Einstechnadel verlaufenden Schneidkante weiter in das Gewebe eingeführt werden. Vorzugsweise ist der Schlauch aus Formgedächtnismaterial in dem ersten Zustand derart in Längsrichtung der Einstechnadel gefaltet, dass die Innenflächen der Wandung des Schlauches aufeinander zu liegen kommen und sich vier kreuzförmig, vorzugsweise im rechten Winkel angeordnete, Falten entlang der Einstechnadellängsachse ausbilden. Grundsätzlich könnte der Schlauch aber auch derart gefaltet sein, dass sich entlang der Längsachse der Einstechnadel drei Falten oder auch fünf Falten oder mehr ausbilden. Falls die Innenflächen des Schlauches aufeinandergefaltet sind, weist die Einstechnadel in dem ersten Zustand keinen inneren Hohlraum auf.

Nach dem Einführen der Einstechnadel wechselt das Formgedächtnismaterial von der ersten Form, die den ersten Zustand bildet, zu einer zweiten Form, die den zweiten Zustand der Einstechnadel bildet. In dieser zweiten Form ist der Umfang des Schlauches, bzw. der Einstechnadel vorzugsweise kantenlos ausgebildet und es entsteht ein entlang der Einstechnadellängsachse am Ende, d. h. ausgebildetes inneres Lumen und eine Öffnung in Richtung der Einstechnadellängsachse am Ende, d. h. an der Spitze der Einstechnadel. Hierfür entfaltet sich z.B. der Schlauch aus Formgedächtnismaterial, so dass die von den Falten gebildeten Kanten geglättet werden. Durch das Entfalten entsteht das innere Lumen entlang der Einstechnadellängsachse, das als Öffnung in Richtung der Längsachse aus dem Ende der Einstechnadel mündet. In diesem zweiten in das Körpergewebe eingeführten, entfalteten Zustand der Einstechnadel kann durch das Innere Lumen und die Öffnung ein Fluid in das Gewebe eingeführt werden.

Bei einer anderen Ausführungsform einer erfindungsgemäßen Einstechnadel weist diese einen elastischen Schlauch, vorzugsweise aus Formgedächtnispolymer, auf, der vorzugsweise auch dehnbar ist. In dem von dem Schlauch gebildeten Innenraum sind steife Fasern, vorzugsweise Kohlefasern oder Glasfasern, entlang der Einstechnadellängsachse angeordnet. In einer ersten Form, dem ersten Zustand der Einstechnadel, ist die Einstechnadel solide, indem die Fasern durch den Schlauch zusammengedrückt werden, und weist vorzugsweise keine Hohlräume im Inneren auf. In dieser kompakten Form ist die Einstechnadel ausreichend steif, weil sich die Fasern gegenseitig versteifen, um in das Gewebe eingeführt werden zu können. Der Schlauch kann auf die Fasern, diese umgebend aufgeschrumpft sein. In einer zweiten Form, das heißt dem zweiten Zustand der Einstechnadel, hat sich der Schlauch ausgedehnt. Die Fasern liegen lose im Schlauch und setzen einem Verbiegen nur noch je die Biegesteifigkeit der Einzelfasern entgegen.

In noch einer anderen Ausführungsform weist die erfindungsgemäße Einstechnadel einen Schlauch auf, der abschnittsweise in seiner Wandung Bereiche aus Formgedächtnismaterial aufweist. Die zwischen den Formgedächtnismaterialbereichen liegenden Abschnitte der Wandung sind vorteilhafterweise steif ausgebildet. Vorzugsweise sind die Bereiche aus Formgedächtnismaterial als Faltung ausgebildet, deren Falten senkrecht zur Einstechnadellängsachse verlaufen, das heißt, der Schlauch wird durch die Faltung verkürzt. In einer ersten Form des Formgedächtnismaterials kommen die Falten aufeinander zu liegen. Die Falten, das heißt die Bereiche aus Formgedächtnismaterial, sind daher in Richtung der Längsachse der Einstechnadel nicht weiter komprimierbar und die Einstechnadel ist derart steif, dass sie in das Gewebe eingestochen werden kann. In einer zweiten Form nimmt das Formgedächtnismaterial eine ausgedehnte Form mit auseinandergezogenen Falten an. In dieser Form sind die Formgedächtnismaterialbereiche entlang der Längsachse der Einstechnadel komprimierbar und expandierbar. Die Einstechnadel wird dadurch in einer Richtung radial zur Einstechnadellängsachse beweglich. Die Formgedächtnismaterialbereiche sind vorzugsweise als Ringbereiche um den Umfang des Einstechnadelschlauches angeordnet. An diesen Ringbereichen kann die Einstechnadel dann in der zweiten Form, das heißt in dem zweiten Zustand, nach dem Einführen in ein Gewebe dessen Bewegungen folgen.

In einer weiteren Ausführungsform ist in einem vorderen Bereich des Schlauches eine Einstechhilfe angeordnet, die im ersten Zustand der Einstechnadel über das Ende, das die Spitze der Einstechnadel bildet, in Einstechnadellängsrichtung hervorsteht. Die Einstechhilfe kann z.B. von einer Nadelspitze gebildet werden, die mit Hilfe einer Verlängerung an einem Befestigungspunkt an der Einstechnadel befestigt ist, der in einem Abstand zur Einstechnadelspitze vorgesehen ist. Das heißt, die Aufhängung der Einstechhilfe liegt nicht am distalen Ende der Einstechnadel, sondern in einem nach proximal davon entfernt liegenden Bereich. In dem ersten Zustand, in dem die Falten der Formgedächtnisbereiche aufeinander zu liegen kommen, ragt die Spitze aus dem Ende des Einstechnadelschlauches hervor. Es sind mehrere wie oben beschrieben ausgebildete Bereiche aus Formgedächtnismaterial zwischen der Spitze der Einstechhilfe und ihrem Befestigungspunkt vorgesehen. In dem zweiten Zustand der Einstechnadel sind diese Bereiche entfaltet, sodass sich der Einstechnadelabschnitt zwischen Befestigungspunkt und Einstechnadelende verlängert. Dabei wird der Einstechnadelschlauch über die Einstechhilfe hinaus verschoben, so dass er als Schutz für die Einstechspitze dienen kann.

Anstelle einer Faltung können die Bereiche aus Formgedächtnismaterial in der Wandung des Schlauches der Einstechnadel natürlich auch eine andere Form aufweisen, solange sie in einem ersten Zustand der Einstechnadel eine ausreichende Steifigkeit und in einem zweiten Zustand der Einstechnadel eine ausreichende Nachgiebigkeit, vorzugsweise Biegsamkeit, aufweisen. Beispielsweise können die Bereiche auch perforiert ausgebildet sein. Als Perforation können z.B. kleine Schlitze in der Wandung des Schlauches vorgesehen sein, die z.B. aufeinanderfolgend in Umfangsrichtung verlaufen und in mehreren Reihen nebeneinander angeordnet sind. Die Schlitze können nur in einer Oberfläche des Schlauches oder auch durchgehend durch die Wandung ausgebildet sein. In einer ersten Form der Formgedächtnismaterialbereiche kommen die Schlitze aufeinander zu liegen. In einer zweiten Form werden die Schlitze aufgeweitet, sodass sich eine Biegsamkeit in radialer Richtung zur Einstechnadellängsachse ergibt.

Bei einer anderen Ausführungsform besteht die Einstechnadel wenigstens abschnittsweise aus zumindest einem Gel. Die Einstechnadel wird vorzugsweise aus einem Schlauch gebildet, der in seiner Wandung wenigstens abschnittsweise einen oder mehrere in Längsrichtung der Einstechnadellängsachse verlaufende Hohlräume aufweist. Der Schlauch besteht daher wenigstens aus einem inneren Lumen zum Durchleiten eines Fluids und innerhalb der Wandung vorgesehenen Hohlräumen. Die Wandungshohlräume sind mit Gel gefüllt. Der Schlauch selbst besteht aus elastischem, biegsamen Material. Es ist auch möglich, das Gel in einer schlauchartigen Hülle vorzusehen, und die Hülle an einer Außen- oder Innenfläche des Einstechnadelschlauches anzuordnen.

In einem ersten Zustand ist das Gel fest. In diesem Zustand bildet es eine Stützstruktur für den Einstechnadelschlauch, der dadurch ausreichend steif ist, um in ein Körpergewebe eingeführt zu werden. In einem zweiten Zustand ist das Gel weich, sodass die Gelhohlräume, bzw. die Gelhülle, elastisch sind und keine Stützwirkung für den Einstechnadelschlauch entsteht.

Aus dem Wechsel zwischen einem ersten festen Zustand zu einem flexiblen, vorzugsweise elastischen, zweiten Zustand des Materials der Einstechnadel ergibt sich eine einfache Handhabung beim Einbringen der Einstechnadel in ein Gewebe, ohne dass hierfür mehrere verschiedene Einzelteile notwendig sind. Die Zustandsänderung zwischen dem ersten und dem zweiten Zustand des Materials der Einstechnadel kann durch ein Zustandsänderungsmittel erfolgen, das z.B. eine elektrische Spannung oder eine Strahlung auf die Einstechnadel, bzw. das erfindungsgemäße Material der Einstechnadel ausübt. Die Zustandsänderung kann auch durch Einwirkung einer chemischen Reaktion, eines Temperaturwechsels oder eines Wechsels des pH-Wertes erfolgen. Dies kann z.B. durch die Änderung der Umgebung der Einstechnadel beim Einbringen in das Gewebe, das heißt durch das Umgebungsmilieu des Gewebes, erfolgen oder durch ein an die Einstechnadel herangeleitetes Fluid erzeugt werden. Vorzugsweise ist die Zustandsänderung reversibel. Das heißt, die Einstechnadel kann von einem festen ersten Zustand in einen elastischen zweiten Zustand und wieder zurück in einen festen ersten Zustand gebracht werden.

Nach einem weiteren Aspekt der vorliegenden Erfindung ist die Einstechnadel bei Einwirkung einer Kraft auf die Einstechnadel in Längsrichtung einer Einstechnadellängsachse steif und bei Einwirkung einer Kraft in radialer Richtung der Einstechnadellängsachse elastisch. Zum Einstechen der Einstechnadel wird diese mit der Einstechnadelspitze auf die Oberfläche eines Körpergewebes aufgesetzt und auf dieses aufgedrückt. Dabei wirkt eine Kraft in Längsrichtung der Einstechnadelachse. Bei Einwirken einer derartigen Kraft ist die Einstechnadel steif und nicht nachgiebig. Innerhalb des Körpergewebes entstehen, z.B. durch Muskelbewegungen des Patienten, seitlich auf die Einstechnadel einwirkende Kräfte, das heißt in radialer Richtung zur Einstechnadellängsachse wirkende Kräfte. Gegenüber dieser Krafteinwirkung verhält sich die Einstechnadel elastisch, das heißt sie ist in dieser Richtung nachgiebig.

In einer Ausführungsform weist die Einstechnadel einen Schlauch auf, dessen Wandung steife Bereiche und abwechselnd hierzu biegsame Bereiche, die ein Abbiegen des Schlauches radial zur Einstechnadellängsachse ermöglichen, umfasst. Die biegsamen Bereiche können z.B. durch eine Faltung der Wandung oder eine Perforation der Wandung gegeben sein. Diese Bereiche können ähnlich ausgebildet sein, wie oben für Bereiche aus Formgedächtnismaterial beschrieben. Die Bereiche müssen jedoch nicht aus Formgedächtnismaterial bestehen. Die Steifigkeit bzw. Biegsamkeit der Einstechnadel ergibt sich bei dieser Ausführungsform aus der besonderen Struktur der Schlauchwandung. Im Falle einer Faltung der Wandung können die gefalteten Bereiche nicht weiter komprimiert werden, wenn die einzelnen Falten aufeinander zu liegen kommen. Beim Einwirken einer entsprechenden Kraft zur Kompression, das heißt, einer Kraft in Längsrichtung der Einstechnadelachse, sind diese Bereiche deshalb steif. Die Faltungen lassen sich jedoch auseinanderziehen. Wird ein ringförmig um den Umfang des Schlauches angeordneter Faltenbereich an einer Seite mehr auseinandergezogen als an einer gegenüberliegenden Seite, ergibt sich eine Biegung des Schlauches, bzw. der Einstechnadel. Die biegsamen und steifen Bereiche der Schlauchwandung wechseln sich derart ab, dass die Einstechnadel in einem in das Gewebe eingebrachten Zustand auf seitliche Krafteinwirkungen biegsam reagiert.

Bei einer anderen Ausführungsform weist die Einstechnadel einen elastischen Schlauch auf, in dessen Innenraum eine Stützstruktur aufgenommen ist. Die Stützstruktur ist in Richtung radial zur Einstechnadellängsachse beweglich und in einer Richtung entlang der Einstechnadellängsachse steif. Als Stützstruktur kann z.B. eine Spiralfeder dienen, deren Windungen in gerade ausgerichtetem Zustand der Spiralfeder aufeinander liegen. Die Spiralfeder ist derart in den elastischen Schlauch eingebracht, dass der Schlauch auf dem Außenumfang der Feder zu liegen kommt. Zwischen den Windungen können auch Abstandshalter vorgesehen sein, durch die die Windungen in einem Abstand aneinander, bzw. an den Abstandshaltern, liegen. Durch das Aufeinandertreffen der Windungen ist die Spiralfeder bei Einwirken einer Kraft in Längsrichtung der Feder, bzw. der Einstechnadel, steif. Bei einer seitlich einwirkenden Kraft in radialer Richtung zur Einstechnadellängsachse können sich die Windungen jedoch an einer Seite voneinander abheben und dadurch eine Biegung der Spiralfeder, bzw. der Einstechnadel, ermöglichen. Vorteilhafterweise entsteht durch die Spiralform der Feder an ihrem Ende eine Einstechspitze zum Einstechen der Einstechnadel in das Gewebe.

Bei noch einer anderen Ausführungsform umfasst eine erfindungsgemäße Einstechnadel einen elastischen Schlauch, der ein inneres Lumen zum Durchleiten eines Fluids, das heißt, einen entlang der Einstechnadellängsachse verlaufenden Innenraum und wenigstens einen in der Wandung des Schlauchs angeordneten Zwischenraum umfasst. In dem Zwischenraum ist erfindungsgemäß ein Füllmaterial vorgesehen, das in einem ersten Zustand durch ein Kompressionsmittel in Richtung der Einstechnadellängsachse komprimiert und in einem zweiten Zustand durch Lösen des Kompressionsmittels entkomprimiert ist. Als Füllmaterial kann z.B. ein Fasermaterial, ein Granulat, ein Gel und/ein Glas dienen. Durch das Komprimieren des Füllmaterials dehnt sich der Zwischenraum senkrecht zur Längsachse der Einstechnadel aus, wodurch er steif wird und als Stütze für die Einstechnadel dient. Dabei dehnt er sich vorzugsweise in Richtung des Innenraums des Schlauches aus, wodurch sich das innere Lumen verengt, jedoch der Gesamtumfang des Schlauches nicht wesentlich vergrößert wird. Durch ein Entkomprimieren des Zwischenraums verteilt sich das Füllmaterial über die vergrößerte Gesamtlänge des Zwischenraums entlang der Längsachse der Einstechnadel und ist im wesentlichen beweglich innerhalb des Zwischenraums angeordnet, sodass die Einstechnadel biegsam wird. Als Kompressionsmittel kann z.B. ein Ring dienen, der um den Schlauch angeordnet ist und entlang der Einstechnadellängsachse verschiebbar ist. Durch das Verschieben des Ringes entlang der Einstechnadellängsachse wird das Füllmaterial im Zwischenraum komprimiert oder entkomprimiert.

Vorzugsweise ist die Außenwandung des elastischen Schlauchs in dieser Ausführungsform weniger dehnbar und die Innenwandung, welche als Zwischenwand zwischen dem Zwischenraum und dem Innenraum dient, sehr dehnbar ausgebildet. Beim Komprimieren des Füllmaterials in dem Zwischenraum, dehnt sich dieser daher hauptsächlich ins Innere des Schlauches aus.

Die vorliegende Erfindung wurde anhand verschiedener Ausführungsbeispiele beschrieben. Die einzelnen Merkmale der einzelnen Ausführungsbeispiele können jedoch vorteilhaft miteinander kombiniert werden, wie z.B. die Ausführungsform einer Einstechnadel mit faltenförmigen Bereichen in der Einstechnadelwandung, die aus Formgedächtnismaterial bestehen, zeigt. Die Erfindung wird nachfolgend anhand von Figuren an verschiedenen Ausführungsbeispielen erläutert. Es zeigen:

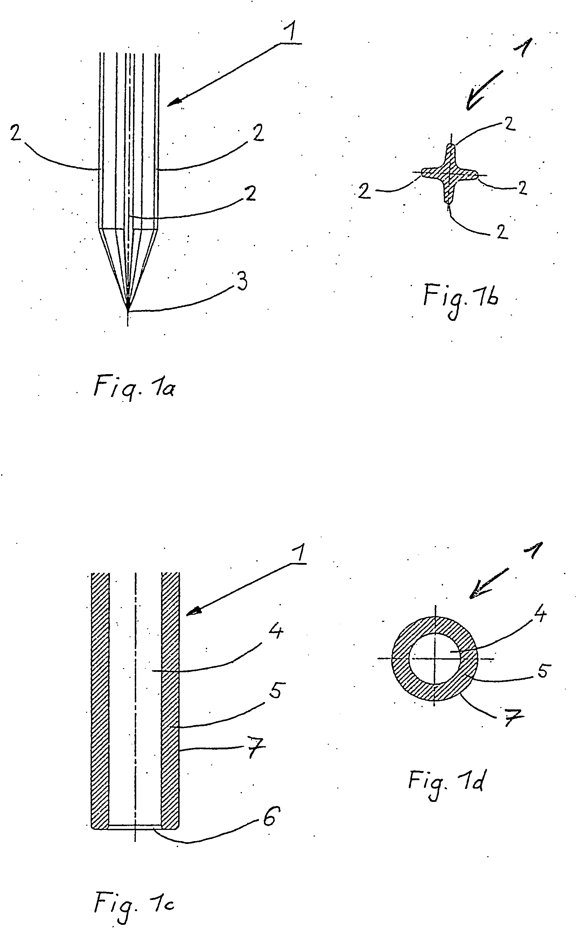

- Fig. 1a: schematische Darstellung einer erfindungsgemäßen Einstechnadel in einer ersten Ausführungsform in einem festen ersten Zustand,
- Fig. 1b: Querschnitt durch die Einstechnadel aus Fig. 1a,
- Fig. 1c: Längsschnitt durch die Einstechnadel nach der ersten Ausführungsform in einem flexiblen zweiten Zustand,
- Fig. 1 d: Querschnitt durch die Einstechnadel nach Fig. 1c,
- Fig. 2a: schematische Darstellung einer erfindungsgemäßen Einstechnadel nach einer zweiten Ausführungsform in einem festen ersten Zustand,
- Fig. 2b: Querschnitt durch die Einstechnadel nach Fig. 2a,
- Fig. 2c: Querschnitt durch die Einstechnadel nach der zweiten Ausführungsform in einem flexiblen zweiten Zustand,
- Fig. 3a: schematischer Längsschnitt durch eine erfindungsgemäße Einstechnadel nach einer dritten Ausführungsform,
- Fig. 3b: schematische Darstellung einer erfindungsgemäßen Einstechnadel nach einer vierten Ausführungsform,
- Fig. 3c: schematische Darstellung einer erfindungs gemäßen Einstechnadel nach der dritten Ausführungsform,
- Fig. 4a: schematische Darstellung einer erfindungsgemäßen Einstechnadel nach einer fünften Ausführungsform in einem festen ersten Zustand,
- Fig. 4b: schematische Darstellung der Einstechnadel nach der fünften Ausführungsform in einem flexiblen zweiten Zustand,
- Fig. 5: Längsschnitt durch eine erfindungsgemäße Einstechnadel nach einer sechsten Ausführungsform,
- Fig. 6a: Längsschnitt durch eine erfindungsgemäße Einstechnadel nach einer siebten Ausführungsform in einem festen ersten Zustand,
- Fig. 6b: Längsschnitt durch eine erfindungsgemässe Einstechnadel nach einer siebten Ausführungsform in einem biegsamen Zustand.
- Fig. 7a: eine erfindungsgemäße Einstechnadel nach einer achten Ausführungsform in einem festen ersten Zustand,
- Fig. 7b: die Einstechnadel der achten Ausführungsform in einem flexiblen zweiten Zustand,
- Fig. 8a: eine erfindungsgemäße Einstechnadel nach einer neunten Ausführungsform in einem festen ersten Zustand und
- Fig. 8b: die Einstechnadel der neunten Ausführungsform in einem flexiblen zweiten Zustand.

In den Figuren 1a bis 1d ist eine erfindungsgemäße Einstechnadel 1 gemäß einer ersten Ausführungsform gezeigt. Die Einstechnadel ist vollständig aus einem Formgedächtnismaterial hergestellt, das in einer schlauchartigen Form bereitgestellt wird. In Fig. 1a und 1b ist die Einstechnadel 1 in einer ersten Form gezeigt, in der sie einen festen, zum Einstechen in ein Gewebe geeigneten Zustand einnimmt. Das Formgedächtnismaterial ist in diesem Zustand wie ein gefalteter Schlauch geformt, so dass sich vier Kanten 2 ergeben. Wie der Fig. 1b zu entnehmen ist, sind in dieser Ausführungsform die vier Kanten kreuzförmig zueinander angeordnet. Im Inneren des schlauchförmigen Formgedächtnismaterials bleibt in der ersten Form kein Hohlraum erhalten. In einer Abwandlung kann jedoch ein Hohlraum im ersten Zustand gebildet sein. Ein Ende des schlauchartigen Formgedächtnismaterials bildet eine Spitze 3 der Einstechnadel 1. An der Spitze 3 laufen die Kanten 2 in einem Punkt zusammen. Es ist denkbar, an der Spitze 3 eine zusätzliche Einführhilfe, in Form eines harten und spitzen Elements, zum Durchstechen der Gewebeoberfläche vorzusehen. In diesem gefalteten festen ersten Zustand stützen sich die gefalteten Kanten 2 gegenseitig ab. Die Einstechnadel weist einen ausreichend steifen Zustand auf, um in ein Körpergewebe eindringen zu können.

In den Fig. 1c und 1d ist die Einstechnadel nach der ersten Ausführungsform in einem flexiblen, zweiten Zustand dargestellt, in dem die Einstechnadel 2 eine gegenüber dem ersten Zustand verringerte Biegesteifigkeit aufweist, so dass sie nachgiebiger, vorzugsweise elastisch nachgiebig, ist. Durch Stimulation des schlauchförmigen Formgedächtnismaterials, wie z.B. durch eine Erwärmung oder durch den Einfall von Strahlung, nimmt das Formgedächtnismaterial seine zweite Form an, in der die Einstechnadel biegsam ist und ein inneres Lumen 4 zum Durchleiten eines Fluids aufweist. Die Einstechnadel nimmt in diesem Zustand die Form eines Schlauches 7 an, in dem sich die Faltung gemäß der ersten Form auflöst und sich der Schlauch 7 mit einer kreisförmigen Wandung 5 ergibt, wie im Querschnitt in Fig. 1d zu sehen. In diesem Zustand weist die Einstechnadel ein offenes Ende als Öffnung 6 auf, durch welche ein Fluid in das Gewebe abgegeben werden kann. Die Biegsamkeit im zweiten Zustand wird durch einen verringerten E-Modul des Nadelmaterials erzielt. Alternativ kann die Querschnittsform und/oder die Abmessung der Einstechnadel im ersten Zustand so gewählt sein, dass sie im ersten Zustand ein größeres Flächenträgheitsmoment als im zweiten Zustand aufweist. Denkbar ist es ferner, dass im ersten Zustand sowohl der E-Modul als auch das Trägheitsmoment größer als im zweiten Zustand sind.

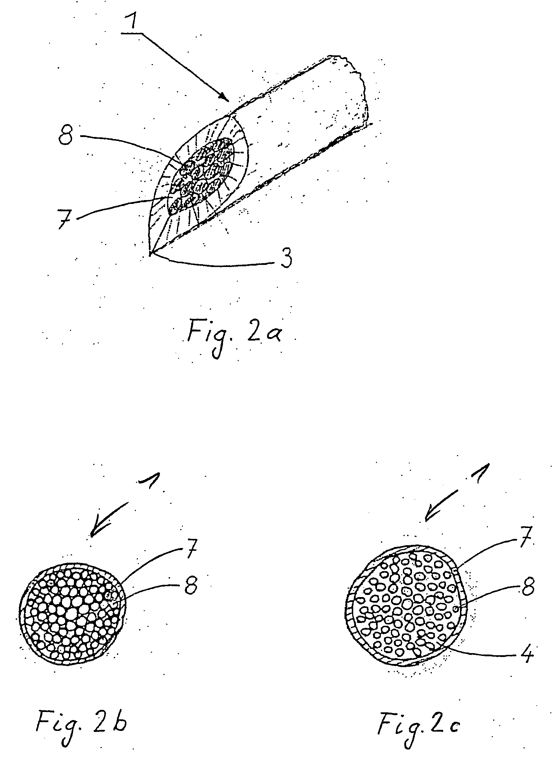

In den Figuren 2a bis 2c ist eine erfindungsgemäße Einstechnadel 1 in einer zweiten Ausführungsform gezeigt. Wie in Fig. 2a dargestellt ist, weist die Einstechnadel 1 einen elastischen Schlauch 7 aus einem Formgedächtnismaterial auf. Der Schlauch 7 umschließt ein Füllmaterial 8 in Form eines Faserbündels, das sich entlang der Längsachse der Einstechnadel 1 erstreckt. In einem ersten Zustand umschließt das Formgedächtnismaterial das Faserbündel 8 fest, so dass die Fasern des Bündels 8 dicht gepackt in dem Schlauch 7 untergebracht sind. Die einzelnen Fasern des Bündels 8 liegen dicht aneinander, so dass sie sich gegenseitig abstützen und im Wesentlichen kein Hohlraum im Inneren des Schlauches 7 verbleibt. Einer Biegung der Einstechnadel 1 wirken im ersten Zustand daher die Reibungskräfte zwischen den Fasern entgegen. In diesem Zustand kann die Einstechnadel 1 in ein Körpergewebe eingestochen werden. An einem vorderen Ende ist der Schlauch 7 mit zwei Phasen angeschrägt, sodass sich eine Spitze 3 zum Einstechen der Einstechnadel in das Gewebe ergibt. In Fig. 2b ist ein Querschnitt der Einstechnadel in dem festen, d. h. biegesteifen, ersten Zustand gezeigt, in welchem die Fasern des Bündels 8 innerhalb des Schlauchs 7 fest aneinander liegen.

Nach dem Einbringen in das Körpergewebe geht der Schlauch 7 durch Stimulation in einen zweiten Zustand über, in welchem die einzelnen Fasern des Bündels 8 nicht mehr oder allenfalls nur noch lose aneinander gedrückt werden, so dass sich zwischen ihnen der Zwischenraum, der als Lumen 4 dient, vergrößert oder erst ergibt. In dieser Form sind die Fasern des aufgelösten Bündels 8 zueinander beweglich und biegbar.

Das Füllmaterial 8 des Schlauchs 7 muss nicht faserförmig sein, obgleich solch ein Füllmaterial bevorzugt wird. Es sind grundsätzlich auch andere geeignete Formen möglich, solange in einem ersten Zustand die Einstechnadel 1 eine ausreichende Steifigkeit zum Einstechen in ein Gewebe und in einem zweiten Zustand eine ausreichende Biegsamkeit aufweist. Beispielsweise könnte das Füllmaterial 8 auch die Form von Kügelchen aufweisen. Der Schlauch 7 würde solch ein Füllmaterial umschließend verpressen.

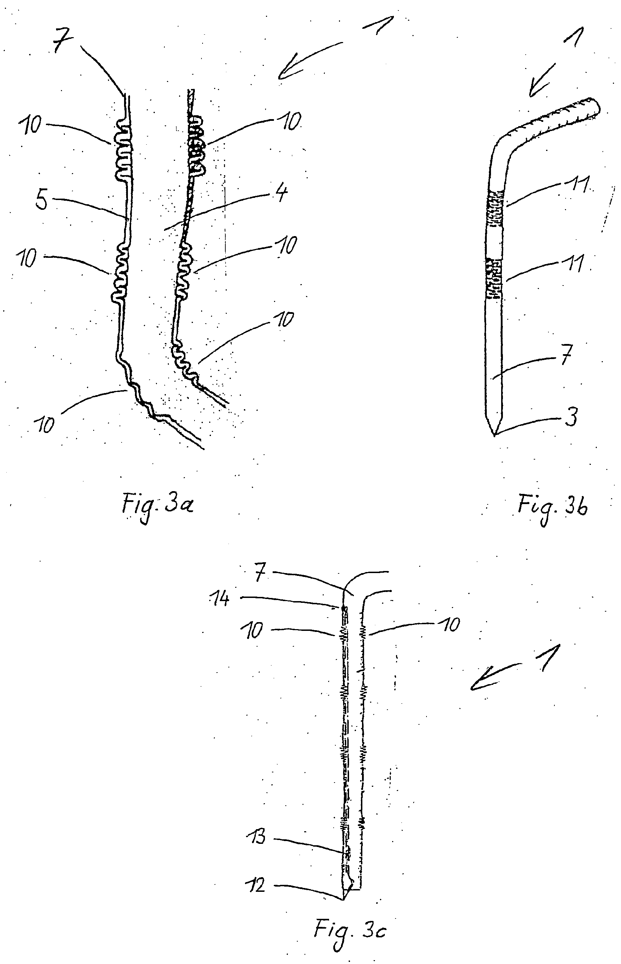

In Fig. 3a ist eine erfindungsgemäße Einstechnadel 1 in einer dritten Ausführungsform gezeigt. Die Einstechnadel 1 ist schlauchartig ausgebildet. Vorteilhafterweise ist sie aus einem Formgedächtnismaterial hergestellt. In der Wandung 5 eines Schlauchs 7 sind entlang der Längsachse der Einstechnadel in regelmäßigen Abschnitten Faltbereiche 10 ausgebildet, in welchen die Wandung 5 in Umfangsrichtung verlaufende Falten aufweist, die in Längsrichtung der Einstechnadellängsachse nebeneinander angeordnet sind. Es ist auch denkbar, nur die Faltbereiche 10 aus Formgedächtnismaterial herzustellen und für die dazwischenliegenden Schlauchabschnitte ein anderes Material zu verwenden.

In einem ersten Zustand, kommen die einzelnen Faltenlagen der Faltbereiche 10 aufeinander zu liegen. Ein weiteres Zusammenpressen der Faltbereiche 10 in Längsrichtung der Einstechnadellängsachse ist nicht möglich. Bei Einwirkung einer Kraft auf die Einstechnadel 1 in Längsrichtung der Einstechnadellängsachse verhält sich die Einstechnadel 1 daher steif. Bei Einwirkung einer Kraft in radialer Richtung zur Einstechnadellängsachse kann die Einstechnadel 1 an den Faltenbereichen 10 gegenüber der Längsachse gebogen werden. Dabei weiten sich die einzelnen Falten der Faltenbereiche 10 auf, wie in Fig. 3a am untersten Faltenbereich 10 dargestellt ist. Falls die Faltenbereiche aus Formgedächtnismaterial hergestellt sind, kann die Steifigkeit bei Einwirken einer Kraft in Längsrichtung durch eine erste Form des Formgedächtnismaterials unterstützt werden, in welcher die Faltung der Faltenbereiche 10 nicht auseinanderziehbar ist. In einer zweiten Form, wenn die Einstechnadel 1 in ein Körpergewebe eingeführt ist, ist das Formgedächtnismaterial an den Faltenbereichen 10 auseinanderfaltbar, so dass die Einstechnadel in eingeführtem Zustand biegsam ist.

In Fig. 3b ist eine vierte Ausführungsform der erfindungsgemäßen Einstechnadel 1 gezeigt, die der dritten Ausführungsform aus Fig. 3a ähnlich ist. Anstelle der Faltenbereiche 10 sind in der vierten Ausführungsform Perforationsbereiche 11 vorgesehen. In den Perforationsbereichen 11 weist eine Wandung eines Schlauches 7 in Umfangsrichtung hintereinander angeordnete kleine Schlitze auf. Einzelne Reihen von Schlitzen sind in Längsrichtung der Einstechnadellängsachse nebeneinander angeordnet, wobei die Schlitze zueinander versetzt sind. Sind die Schlitze durchgehend durch die Wandung vorgesehen, kann im Inneren des Schlauches z.B. eine dünne elastische Haut vorgesehen sein, so dass durch die Schlitze kein Fluid aus der Einstechnadel austritt. Eine solche Haut kann jedoch auch weggelassen werden, wenn z.B. das Fluid großflächig und nicht punktgenau innerhalb des Gewebes abgegeben werden soll. Die Schlitze können aber auch derart ausgebildet sein, dass sie weit in eine Innen- oder Außenoberfläche des Schlauches hineinreichen, jedoch nicht durch die Wandung des Schlauches hindurchgehen.

Beim Einwirken einer Kraft auf die Einstechnadel 1 in Längsrichtung liegen die Schlitze dicht aufeinander, so dass die Einstechnadel ausreichend steif ist, um in ein Körpergewebe eingestochen zu werden. Beim Einwirken einer Kraft in radialer Richtung zur Einstechnadelachse öffnen sich die Schlitze der Perforationsbereiche 11 an einer Seite, so dass die Einstechnadel zur gegenüberliegenden Seite gegenüber der Einstechnadellängsachse biegsam ist. Die Perforation der Perforationsbereiche 11 kann viele verschiedene Formen annehmen, beispielsweise kann sie auch wellenförmig sein.

In Fig. 3c ist eine Einstechnadel 1 nach der dritten Ausführungsform gemäß der Fig. 3a gezeigt, die zusätzlich eine Einstechhilfe 12 aufweist. Der Schlauch 7 der Einstechnadel weist wie oben beschrieben abschnittsweise Faltenbereiche 10 auf. Die Einstechhilfe 12 umfasst eine Einstechspitze 12, die an einer Verlängerung 13 angeordnet ist. Die Verlängerung 13 ist derart im Inneren des Schlauches 7 angeordnet, dass die Einstechspitze 12 in einem ersten Zustand der Einstechnadel aus dem Ende des Schlauches 7 herausragt. Die Verlängerung 13 ist im Inneren des Schlauches 7 an einem Befestigungspunkt 14 befestigt. Der Befestigungspunkt 14 liegt an einer Stelle innerhalb des Schlauchs 7, sodass sich mehrere Faltbereiche 10 zwischen dem Befestigungspunkt 14 und dem Ende des Schlauchs 7 befinden. In einem zweiten Zustand sind die Falten der Faltenbereiche 10 des Schlauches 7 aufgefaltet, das heißt, auseinandergezogen, sodass sich die Strecke vom Befestigungspunkt 14 bis zum Ende des Schlauches verlängert. Der Schlauch schiebt sich dabei im zweiten Zustand über die Einstechspitze 12 der Einstechhilfe. In diesem Zustand bildet sie einen Schutz vor Verletzungen an der Einstechspitze 12.

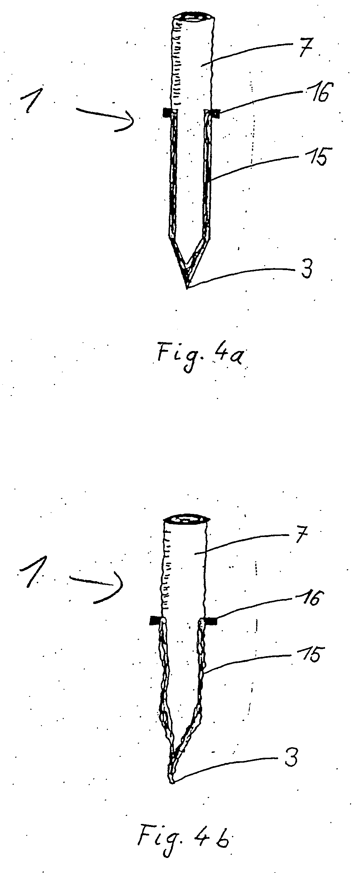

In den Figuren 4a und 4b ist eine fünfte Ausführungsform einer erfindungsgemäßen Einstechnadel 1 gezeigt. Die Einstechnadel weist einen Schlauch 7 auf, der an einem zum Einstechen in ein Körpergewebe vorgesehenen Ende in seiner Wandung einen Hohlraum 15 aufweist, der sich in Umfangsrichtung um den gesamten Umfang der Wandung und in Längsrichtung im Wesentlichen über die gesamte Länge der Einstechnadel erstreckt, mit der die Einstechnadel in ein Gewebe eingeführt werden soll. Der Hohlraum 15 ist mit einem Gel befüllt. Das Gel weist in einem ersten Zustand eine feste Form auf, sodass die Einstechnadel 1 ausreichend steif ist, um in ein Körpergewebe eingeführt zu werden. Durch Stimulation des Gels, wie etwa der Temperatur oder einem Wechsel des pH-Wertes, nimmt das Gel einen weichen Zustand ein, sodass die Einstechnadel 1 biegsam ist. Am Ende des Hohlraums 15, bzw. des Schlauches 7, ist der Hohlraum, bzw. der Schlauch spitz zulaufend ausgebildet, um eine Spitze 3 zum Einstechen in das Gewebe auszubilden. An dem der Spitze 3 gegenüberliegenden Ende kann an dem Hohlraum 15 ein Schaltelement 16 angeordnet sein, durch das z.B. eine Stimulation des Gels zum Auslösen einer Zustandsänderung vorgesehen sein kann. Das Schaltelement 16 kann einen beim Einstechen insbesondere durch mechanischen Druck oder anderweitig auslösbaren Schalter bilden, dessen Auslösung die Zustandsänderung des Gels bewirkt.

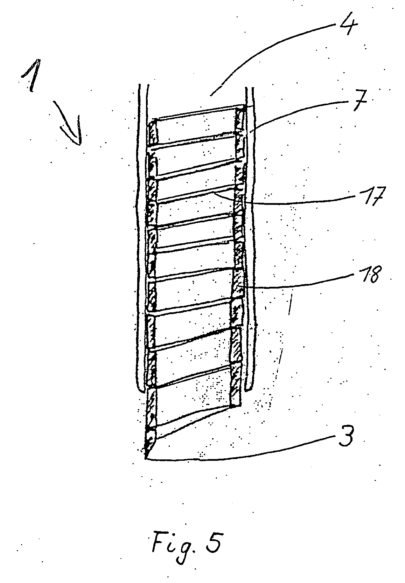

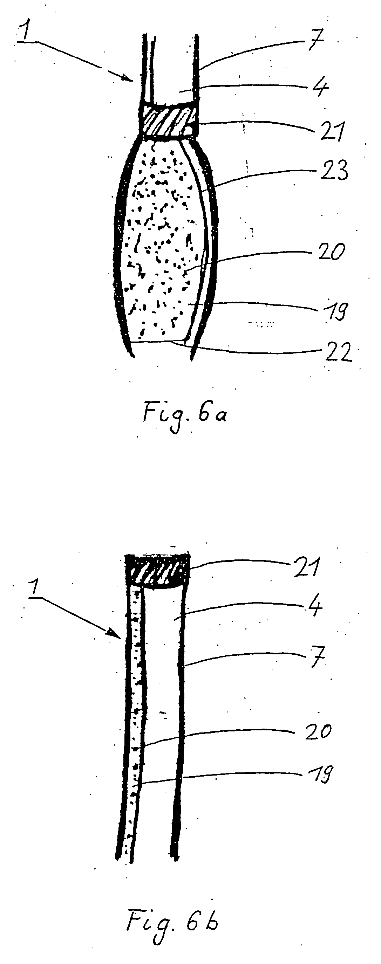

In Fig. 5 ist eine sechste Ausführungsform einer erfindungsgemäßen Einstechnadel 1 gezeigt. Die Einstechnadel 1 umfasst einen Schlauch 7, in dessen innerem Lumen 4 eine Stützstruktur 17 in Form einer Spiralfeder 17 aufgenommen ist. Zwischen den einzelnen Windungen der Spiralfeder 17 sind im wesentlichen radial erstreckte Abstandshalter 18 angeordnet. Die einzelnen Windungen liegen an den Rändern der Abstandshalter 18 an. Beim Einwirken einer Kraft auf die Einstechnadel in Längsrichtung werden die einzelnen Abstandshalter 18 in dieser Richtung aufeinander gedrückt. Die Abstandshalter 18 sind unelastisch ausgebildet, sodass die Einstechnadel beim Einwirken der Kraft in Längsrichtung steif ist und in ein Gewebe eingestochen werden kann. Beim Einwirken einer Kraft in radialer Richtung zur Einstechnadellängsachse von einer Seite der Einstechnadel bewegen sich die Windungen der Spiralfeder 17 auf der gegenüberliegenden Seite auseinander. Dabei wird der Abstand der Windungen auf dieser Seite größer als die Länge der Abstandshalter 18. Auf der anderen Seite bleiben die Windungen an den Abstandshaltern 18 weiterhin anliegend. Beim Einwirken einer Kraft von der Seite auf die Einstechnadel, kann die Einstechnadel daher durch das Aufspreizen der Windungen der Spiralfeder 17 dieser Kraft nachgeben.

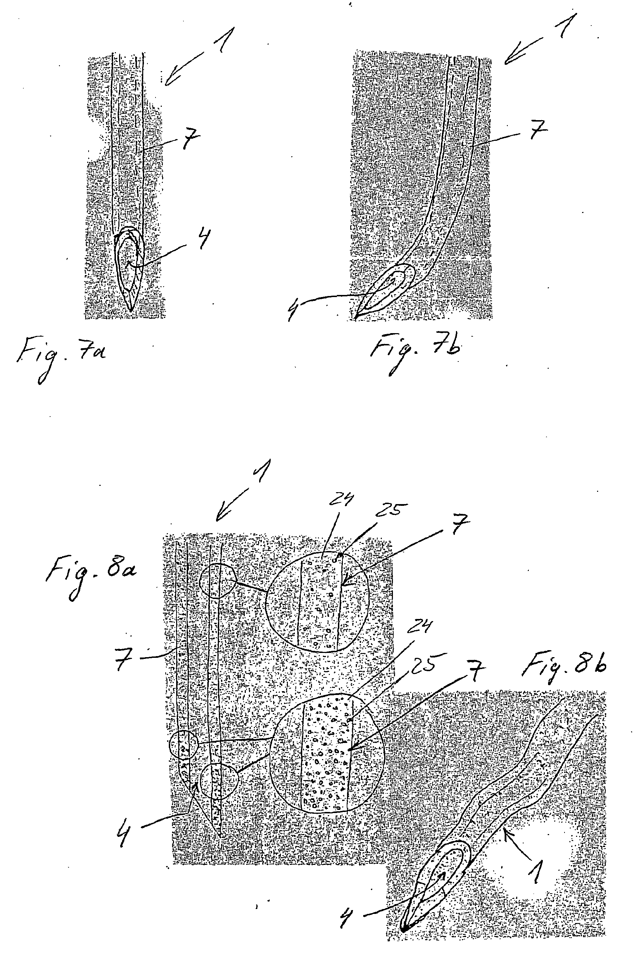

Die Figuren 6a und 6b zeigen eine siebte Ausführungsform einer erfindungsgemäßen Einstechnadel 1. Die Einstechnadel 1 besteht aus einem Schlauch 7, der einen sich in Längsrichtung der Einstechnadellängsachse erstreckenden Zwischenraum 19 in seiner Wandung aufweist, der durch eine Zwischenwand 23 vom Lumen 4 getrennt ist. Der Zwischenraum 19 erstreckt sich vorzugsweise in Umfangsrichtung nicht vollständig um den Umfang des Schlauches 7. In dem Zwischenraum 19 ist ein Füllmaterial 20 vorgesehen, wie z.B. ein Granulat.

In Fig. 6a ist die Einstechnadel 1 in einem ersten Zustand gezeigt, in dem ein Ring 21, der um den Schlauch 7 der Einstechnadel 1 angeordnet ist, das Granulat 20 in dem Zwischenraum 19 komprimiert. Hierfür wird der Ring 21 entlang dem Schlauch 7 derart verschoben, dass das Füllmaterial 20 in Richtung auf einen Abschluss 22 des Zwischenraumes 19 gepresst wird. Die Zwischenwand 23 zwischen dem Zwischenraum 19 und dem Lumen 4 zum Durchleiten eines Fluids ist vorzugsweise elastischer ausgebildet als das Material des Schlauches 7. Durch das Komprimieren des Füllmaterials beim Verschieben des Ringes 21 auf dem Schlauch 7 wird daher die Zwischenwand 23 in Richtung des Lumens und der gegenüberliegenden Schlauchinnenwand ausgedehnt, bis sie an der gegenüberliegenden Schlauchinnenwand zu liegen kommt. In dem komprimierten Zustand des Füllmaterials nimmt dieses eine dicht gepresst feste Form an, so dass es eine versteifende Wirkung auf den Schlauch 7 der Einstechnadel 1 hat. Die Einstechnadel kann in diesem Zustand in ein Körpergewebe eingebracht werden.

In Fig. 6b ist der Ring 21 entlang dem Schlauch 7 zurückverschoben, das heißt, entfernter von dem Abschluss 22. Das Füllmaterial 20 verteilt sich in diesem Zustand über eine größere Länge innerhalb des Zwischenraums 19, so dass sich die Zwischenwand 23 zurück bewegt und das Lumen 4 zum Durchleiten eines Fluids freigibt. Dabei bildet sich ein geringer Abstand zwischen Schlauchinnenwand und Zwischenwand, zwischen welchen das Füllmaterial 20 angeordnet ist, so dass sich nur eine dünne Materialschicht ergibt. In diesem zweiten Zustand, in dem der Ring 21 das Füllmaterial entkomprimiert hat, ist das Füllmaterial 20 im Zwischenraum 19 gegeneinander beweglich und die Einstechnadel ist biegsam. Grundsätzlich ist es auch möglich, anstelle eines einzigen Zwischenraums mehrere aufeinander abgestimmte Zwischenräume entlang der Längsachse der Einstechnadel, bzw. des Schlauches 7, vorzusehen. Ferner kann das Füllmaterial z.B. aus einem Formgedächtnismaterial bestehen, ähnlich wie es bei der zweiten Ausführungsform beschrieben ist. Die Figuren 7a und 7b zeigen eine Einstechnadel 1 in einer achten Ausführungsform. Die Einstechnadel 1 ist über ihre gesamte, in das Gewebe einzustechenden Länge aus einem homogenen Formgedächtnismaterial gefertigt. Sie ist im ersten Zustand, der in Figur 7a dargestellt ist, gerade und weist eine für das Einstechen in das Gewebe ausreichende Biegesteifigkeit auf. Sie kann insbesondere aus einem thermoplastischen Polymer gefertigt sein. Figur 7b zeigt die Einstechnadel 1 der achten Ausführungsform nach dem Einführen in das Gewebe. Aufgrund der im Gewebe herrschenden Verhältnisse ist das Material der Einstechnadel 1 aus dem ersten Zustand in den zweiten Zustand übergegangen, in dem sie so biegeweich ist, dass sie sich unter den im Gewebe auftretenden Belastungen elastisch verbiegt. Das Material der achten Ausführungsform verringert unter den im Gewebe herrschenden Bedingungen seinen E-Modul. Unter Umgebungsbedingungen außerhalb des Gewebes weist es einen E-Modul von vorzugsweise wenigstens 3000 MPa auf. Im eingeführten Zustand weist es hingegen nur noch einen E-Modul von höchstens 1000 MPa, beispielsweise 700 MPa, auf. Die Zustandsänderung kann insbesondere durch einen Übergang des Polymers in den amorphen Zustand verwirklicht sein. Die Zustandsänderung wird bevorzugt durch die Änderung der Temperatur bewirkt, indem das Polymer eine Glasübergangstemperatur aufweist, die bei oder vorzugsweise geringfügig unter der Gewebetemperatur, aber über den üblichen Umgebungstemperaturen liegt. Die Glasübergangstemperatur sollte daher wenigstens 30 °C, vorzugsweise wenigstens 32 °C, betragen und höchstens der Körpertemperatur entsprechen, vorzugsweise höchstens 36°C betragen. Die Einstechnadel 1 ist mit einer Spitze versehen, die eine scharfe Schneidkante bildet. Vorteilhafterweise wird auch diese Spitze im eingeführten Zustand so weich, dass sie nicht mehr schneiden kann. Die Einstechnadel 1 der achten Ausführungsform ist im ersten und im zweiten Zustand eine Einstechkanüle, d. h. sie weist in beiden Zuständen ein inneres Lumen 4 für die Durchleitung des zu verabreichenden Fluids auf.

Die Figuren 8a und 8b zeigen eine Einstechnadel 1 in einer neunten Ausführungsform, die ebenfalls in beiden Zuständen eine Kanüle ist. In der neunten Ausführungsform ist die Einstechnadel 1 über ihre gesamte Länge aus einem Verbundmaterial als Schlauch 7 gebildet. Den Verbund bilden eine Stützmatrix aus einem ersten Material 24 und in der Stützmatrix eingebettete Feststoffkörner 25. Der Volumenanteil der Feststoffkörner 25 verringert sich von der Spitze der Einstechnadel 1 bis zu deren proximalen Ende kontinuierlich. Unmittelbar an der an der Spitze gebildeten Schneidkante beträgt der Volumenanteil des Füllmaterials 25 etwa 70 % und verringert sich bis zum proximalen Ende der Einstechnadel 1, d. h. über die in das Gewebe einzuführende Länge der Einstechnadel 1, bis auf unter 20 %, vorzugsweise auf 10 bis 15 %. Das Material der Stützmatrix 24 ist wieder ein Material, dessen E-Modul im eingeführten Zustand kleiner als vor dem Einstechen ist. Insbesondere kann ein thermoplastisches Polymer das Stützmatrixmaterial bilden, wobei für das Polymer vorzugsweise die Ausführungen zur achten Ausführungsform gelten. Der Schlauch 7 ist außerhalb des Körpers unter Umgebungsbedingungen steif und kann in diesem ersten Zustand im Hinblick auf das Einstechen als Rohr angesehen werden. Im zweiten Zustand ist er elastisch nachgiebig.

### Bezugszeichen:

- 1: Einstechnadellängsachse
- 2: Kante
- 3: Spitze
- 4: Lumen
- 5: Wandung
- 6: Öffnung
- 7: Schlauch, Formgedächtnismaterial
- 8: Füllmaterial
- 9: ---
- 10: Faltbereich
- 11: Perforationsbereich
- 12: Einstechspitze
- 13: Verlängerung
- 14: Befestigungspunkt
- 15: Hohlraum
- 16: Schaltelement
- 17: Spiralfeder
- 18: Abstandshalter
- 19: Zwischenraum
- 20: Füllmaterial
- 21: Ring
- 22: Abschluss
- 23: Zwischenwand
- 24: Stützmatrix
- 25: Füllmaterial

## Patentansprüche

1. Einstechnadel zum Einstechen in ein Körpergewebe, die in eingeführtem Zustand biegsam ist und ein Lumen zum Einleiten eines Fluids ausweist, **dadurch gekennzeichnet, dass**
die Einstechnadel (1) wenigstens abschnittsweise aus zumindest einem Material (7) besteht, das in einem ersten Zustand eine für das Einstechen ausreichende Biegesteifigkeit und in einem zweiten Zustand eine verringerte Biegesteifigkeit aufweist und
die Einstechnadel (1) bei Einwirkung einer Kraft auf die Einstechnadel (1) in Längsrichtung steif ist und bei Einwirkung einer Kraft in radialer Richtung zur Einstechnadellängsachse nachgiebig ist.

2. Einstechnadel nach Anspruch 1, wobei die Einstechnadel (1) wenigstens abschnittsweise aus Formgedächtnismaterial (7) besteht.

3. Einstechnadel nach Anspruch 1, wobei die Einstechnadel (1) über ihre gesamte Länge aus Formgedächtnismaterial (7) besteht.

4. Einstechnadel nach einem der vorhergehenden Ansprüche, wobei das den Zustand ändernde Material (7) in dem ersten Zustand einen E-Modul von über 1000 MPa und in dem zweiten Zustand einen E-Modul von unter 1000 MPa aufweist.

5. Einstechnadel nach dem vorhergehenden Anspruch, wobei das seinen Zustand ändernde Material (7) in dem ersten Zustand einen E-Modul von wenigstens 3000 MPa und in dem zweiten Zustand einen E-Modul von höchstens 800 MPa aufweist.

6. Einstechnadel nach einem der vorhergehenden Ansprüche, wobei das den Zustand ändernde Material (7) ein Verbundmaterial (7) ist und eine Stützmatrix (24) aus einem ersten Material und ein in der Stützmatrix eingebettetes Füllmaterial (25) das Verbundmaterial (7) bilden.

7. Einstechnadel nach dem vorhergehenden Anspruch, wobei Feststoffkörner oder/und ein Fasermaterial das Füllmaterial (25) bilden oder bildet.

8. Einstechnadel nach einem der zwei vorhergehenden Ansprüche, wobei sich der Volumenanteil des Füllmaterials (25) über die Länge der Einstechnadel (1) ändert, vorzugsweise von einer Spitze der Einstechnadel (1) nach proximal verringert.

9. Einstechnadel nach einem der vorhergehenden Ansprüche, wobei das seinen Zustand ändernde Material (7) ein Kunststoffmaterial mit einer Schalttemperatur, vorzugsweise Glasübergangstemperatur oder Schmelztemperatur, unterhalb der Körpertemperatur und oberhalb der Umgebungstemperatur ist oder umfasst, wobei die Glasübergangstemperatur vorzugsweise zwischen 32 °C und 36°C beträgt.

10. Einstechnadel nach einem der vorhergehenden Ansprüche, wobei die Einstechnadel (1) in dem ersten Zustand an ihrem Umfang wenigstens eine sich in Längsrichtung erstreckende Kante (2) aufweist und in dem zweiten Zustand einen kantenlosen Umfang aufweist.

11. Einstechnadel nach einem der vorhergehenden Ansprüche, wobei die Einstechnadel (1) im ersten Zustand ohne einen inneren Hohlraum und im zweiten Zustand entlang der Einstechnadellängsachse mit einem inneren Lumen (4) ausgebildet ist.

12. Einstechnadel nach einem der vorhergehenden Ansprüche, wobei die Einstechnadel (1) im ersten Zustand eine geschlossene Spitze (3) und im zweiten Zustand eine Öffnung (6) an der Spitze (3) aufweist.

13. Einstechnadel nach einem der vorhergehenden Ansprüche, wobei die Einstechnadel (1) einen vorzugsweise elastischen Schlauch (7) aus einem Formgedächtnismaterial umfasst, in dessen Innenraum ein im ersten Zustand mittels des Schlauches (7) dicht gepacktes Faserbündel (8) angeordnet ist.

14. Einstechnadel nach einem der vorhergehenden Ansprüche, wobei die Einstechnadel (1) einen Schlauch (7) umfasst, der in seiner Wandung (5) abschnittsweise Bereiche (10) aus Formgedächtnismaterial aufweist.

15. Einstechnadel nach einem der vorhergehenden Ansprüche, wobei die Einstechnadel (1) einen Schlauch (7) umfasst und in der Wandung (5) des Schlauches (7) Faltbereiche (10) ausgebildet sind, deren Faltenlagen aufeinanderliegen.

16. Einstechnadel nach einem der vorhergehenden Ansprüche, wobei die Einstechnadel (1) wenigstens abschnittsweise mit einem Gel gebildet ist.

17. Einstechnadel nach dem vorhergehenden Anspruch, wobei die Einstechnadel (1) einen Schlauch (7) umfasst, der in seiner Wandung (5) wenigstens abschnittsweise einen oder mehrere in Längsrichtung der Einstechnadel verlaufende Hohlräume (15) aufweist, die mit dem Gel gefüllt sind.

18. Einstechnadel nach einem der vorhergehenden Ansprüche, wobei eine Zustandsänderung vom ersten in den zweiten Zustand durch ein Zustandsänderungsmittel erfolgt, das eine elektrische Spannung und/oder eine Strahlung auf die Einstechnadel ausübt.

19. Einstechnadel nach einem der vorhergehenden Ansprüche, wobei die Zustandsänderung durch Einwirkung einer chemischen Reaktion, eines Temperaturwechsels oder eines Wechsels des pH-Wertes auf die Einstechnadel gegeben ist.

20. Einstechnadel nach einem der vorhergehenden Ansprüche, wobei die Zustandsänderung reversibel ist.

21. Einstechnadel nach einem der vorhergehenden Ansprüche, wobei im ersten Zustand an der Spitze (3) der Einstechnadel eine Einstechhilfe (12; 13) in Einstechnadellängsrichtung über ein Ende der Einstechnadel (1) hervorsteht und im zweiten Zustand das Ende der Einstechnadel (1) über die Einstechhilfe (12; 13) in Einstechnadellängsrichtung hinaus verschoben ist.

22. Einstechnadel nach einem der vorhergehenden Ansprüche, wobei die Einstechnadel einen Schlauch (7) aufweist, dessen Wandung (5) steife Bereiche und radial zur Einstechnadellängsrichtung biegsame Bereiche (10; 11) umfasst.

23. Einstechnadel nach dem vorhergehenden Anspruch, wobei die biegsamen Bereiche (10) eine Faltung der Wandung umfassen.

24. Einstechnadel nach einem der zwei vorhergehenden Ansprüche, wobei die biegsamen Bereiche (11) eine Perforation der Wandung aufweisen.

25. Einstechnadel nach einem der vorhergehenden Ansprüche, wobei die Einstechnadel (1) einen elastischen Schlauch (7) aufweist, in dessen Lumen (4) eine Stützstruktur (17) aufgenommen ist, die in einer Richtung entlang der Einstechnadellängsachse steif ist und in einer Richtung radial zur Einstechnadellängsachse beweglich ist.

26. Einstechnadel nach dem vorhergehenden Anspruch, wobei die Stützstruktur von einer Spiralfeder (17) gebildet wird, deren Windungen aufeinander zu liegen kommen.

27. Einstechnadel nach einem der vorhergehenden Ansprüche, wobei die Einstechnadel (1) einen elastischen Schlauch (7) aufweist, der das Lumen (4) und wenigstens einen in der Wandung (5) angeordneten Zwischenraum (19) umfasst, wobei in dem Zwischenraum (19) ein Füllmaterial (20) vorgesehen ist, das in einem ersten Zustand durch ein Kompressionsmittel (21) komprimiert und in einem zweiten Zustand durch Lösen des Kompressionsmittels entkomprimiert ist.

28. Einstechnadel nach dem vorhergehenden Anspruch, wobei das Kompressionsmittel ein entlang der Einstechnadellängsachse verschiebbarer und um den elastischen Schlauch (7) angeordneter Ring (21) ist.

29. Einstechnadel nach einem der zwei vorhergehenden Ansprüche, wobei das Füllmaterial (20) ein Fasermaterial, ein Granulat oder Partikelmaterial, ein Gel und/oder ein Glas ist oder umfasst.

## Claims

1. Puncture needle for piercing into a body tissue, which is flexible in the inserted state and has a lumen for the introduction of a fluid, **characterized in that**
the puncture needle (1) consists at least section-wise of at least one material (7) which, in a first state, has a bending strength that is adequate for the piercing and, in a second state, has a reduced bending strength and the puncture needle (1) is rigid when a force acts on the puncture needle (1) in longitudinal direction and is non-rigid when a force acts in radial direction relative to the puncture needle longitudinal axis.

2. Puncture needle according to claim 1, wherein the puncture needle (1) consists at least section-wise of shape-memory material (7).

3. Puncture needle according to claim 1, wherein the puncture needle (1) over its whole length consists of shape-memory material (7).

4. Puncture needle according to one of the preceding claims, wherein the material (7) changing the state has a modulus of elasticity of more than 1000 MPa in the first state and a modulus of elasticity of less than 1000 MPa in the second state.

5. Puncture needle according to the preceding claim, wherein the material (7) changing its state has a modulus of elasticity of at least 3000 MPa in the first state and a modulus of elasticity of, at most, 800 MPa in the second state.

6. Puncture needle according to one of the preceding claims, wherein the material (7) changing the state is a composite material (7) and a supporting matrix (24) of a first material and a filling material (25) embedded in the supporting matrix form the composite material (7).

7. Puncture needle according to the preceding claim, wherein solid grains and/or a fibre material form or forms the filling material (25).

8. Puncture needle according to one of the two preceding claims, wherein the proportion by volume of the filling material (25) changes over the length of the puncture needle (1), preferably reducing in proximal direction from a tip of the puncture needle (1).

9. Puncture needle according to one of the preceding claims, wherein the material (7) changing its state is or contains a plastic material with a switching temperature, preferably glass-transition point or melting point temperature, below the body temperature and above the ambient temperature, wherein the glass-transition point temperature is preferably between 32°C and 36°C.

10. Puncture needle according to one of the preceding claims, wherein, in the first state, the puncture needle (1) has at its periphery at least one edge (2) extending in longitudinal direction and, in the second state, has an edgeless periphery.

11. Puncture needle according to one of the preceding claims, wherein, in the first state, the puncture needle (1) is formed without an inner cavity and, in the second state, is formed with an inner lumen (4) along the longitudinal axis of the puncture needle.

12. Puncture needle according to one of the preceding claims, wherein, in the first state, the puncture needle (1) has a sealed tip (3) and, in the second state, has an opening (6) at the tip (3).

13. Puncture needle according to one of the preceding claims, wherein the puncture needle (1) comprises a preferably elastic tube (7) of a shape-memory material, in the interior of which is arranged a fibre bundle (8) tightly packed by means of the tube (7) in the first state.

14. Puncture needle according to one of the preceding claims, wherein the puncture needle (1) comprises a tube (7) which has section-wise in its wall (5) regions (10) of a shape-memory material.

15. Puncture needle according to one of the preceding claims, wherein the puncture needle (1) comprises a tube (7) and folding regions (10), the folded layers of which lie on one another, are formed in the wall (5) of the tube (7).

16. Puncture needle according to one of the preceding claims, wherein the puncture needle (1) is formed at least section-wise with a gel.

17. Puncture needle according to the preceding claim, wherein the puncture needle (1) comprises a tube (7) which has at least section-wise in its wall (5) one or more cavities (15) running in longitudinal direction of the puncture needle, that are filled with the gel.

18. Puncture needle according to one of the preceding claims, wherein a change in state from the first into the second state takes place through a state-changing means that applies an electric voltage and/or a radiation to the puncture needle.

19. Puncture needle according to one of the preceding claims, wherein the change in state is due to the action on the puncture needle of a chemical reaction, a temperature change or a change of pH-value.

20. Puncture needle according to one of the preceding claims, wherein the change in state is reversible.

21. Puncture needle according to one of the preceding claims, wherein, in the first state, at the tip (3) of the puncture needle a puncturing aid (12;13) projects in longitudinal direction of the puncture needle over one end of the puncture needle (1) and, in the second state, the end of the puncture needle (1) is pushed out over the puncturing aid (12;13) in longitudinal direction of the puncture needle (1).

22. Puncture needle according to one of the preceding claims, wherein the puncture needle has tube (7), the wall (5) of which has rigid regions and regions (10; 11) flexible radially to the longitudinal direction of the puncture needle.

23. Puncture needle according to one of the preceding claims, wherein the flexible regions (10) comprise a folding of the wall.

24. Puncture needle according to one of the two preceding claims, wherein the flexible regions (10) contain a perforation of the wall.

25. Puncture needle according to one of the preceding claims, wherein the puncture needle (1) has an elastic tube (7), in the lumen (4) of which is accommodated a supporting structure (17) which is rigid in a direction along the longitudinal axis of the puncture needle and movable in a direction radial to the longitudinal axis of the puncture needle.

26. Puncture needle according to the preceding claim, wherein the supporting structure is formed of a spiral spring (17), the coils of which come to lie on top of each other.

27. Puncture needle according one of the preceding claims, wherein the puncture needle (1) has an elastic tube (7) which contains the lumen (4) and at least one interstice (19) arranged in the wall (5), wherein there is provided in the interstice (19) a filling material (20) which, in a first state, is compressed by a compression means (21) and, in a second state, is decompressed by loosening the compression means.

28. Puncture needle according to the preceding claim, wherein the compression means is a ring (21) that can be moved along the longitudinal axis of the puncture needle and is arranged around the elastic tube (7).

29. Puncture needle according to one of the two preceding claims, wherein the filling material (20) is or contains a fibre material, granular material or particulate material, a gel and/or a glass.

## Revendications

1. Aiguille à piquer destinée à faire des piqûres dans un tissu corporel, qui est souple dans une situation introduite et qui présente un lumen pour l'injection d'un fluide, **caractérisée en ce que**
l'aiguille à piquer (1) est constituée au moins par tronçons en au moins un matériau (7) qui présente, dans un premier état, une raideur en flexion suffisante pour la piqûre et, dans un deuxième état, une raideur réduite en flexion, et
lors de l'application d'une force en direction longitudinale sur l'aiguille à piquer (1), l'aiguille à piquer est rigide, et lors de l'application d'une force en direction radiale par rapport à l'axe longitudinal de l'aiguille à piquer, l'aiguille à piquer est souple.

2. Aiguille à piquer selon la revendication 1, dans laquelle l'aiguille à piquer (1) est constituée au moins par tronçons en un matériau à mémoire de forme (7).

3. Aiguille à piquer selon la revendication 1, dans laquelle l'aiguille à piquer (1) est constituée sur la totalité de sa longueur en un matériau à mémoire de forme (7).

4. Aiguille à piquer selon l'une des revendications précédentes, dans laquelle le matériau (7) changeant d'état présente, dans le premier état, un module E supérieur à 1000 MPa et, dans le deuxième état, un module E inférieur à 1000 MPa.

5. Aiguille à piquer selon la revendication précédente, dans laquelle le matériau (7) changeant d'état présente dans le premier état, un module E d'au moins 3000 MPa et dans le deuxième état, un module E au maximum de 800 MPa.

6. Aiguille à piquer selon l'une des revendications précédentes, dans laquelle le matériau (7) changeant d'état est un matériau composite (7), et une matrice de soutien (24) en un premier matériau et un matériau de remplissage (25) intégré dans la matrice de soutien forment le matériau composite (7).

7. Aiguille à piquer selon la revendication précédente, dans laquelle un matériau solide en grains et/ou un matériau en fibres constitue le matériau de remplissage (25).

8. Aiguille à piquer selon l'une des deux revendications précédentes, dans laquelle la part en volume du matériau de remplissage (25) varie sur la longueur de l'aiguille à piquer (1), de préférence en diminuant depuis une pointe de l'aiguille à piquer (1) en direction proximale.

9. Aiguille à piquer selon l'une des revendications précédentes, dans laquelle le matériau (7) changeant d'état est ou contient une matière plastique avec une température de transition, de préférence une température de transition vitreuse ou une température de fusion, au-dessous de la température corporelle et au-dessus de la température ambiante, la température de transition vitreuse étant de préférence entre 32°C et 36 °C.

10. Aiguille à piquer selon l'une des revendications précédentes, dans laquelle l'aiguille à piquer (1) présente, dans le premier état, sur sa périphérie au moins une arête (2) qui s'étend en direction longitudinale, et présente, dans le deuxième état, une périphérie dépourvue d'arête.

11. Aiguille à piquer selon l'une des revendications précédentes, dans laquelle l'aiguille à piquer (1) est réalisée sans cavité intérieure, dans le premier état, et avec un lumen intérieur (4) le long de l'axe longitudinal de l'aiguille à piquer, dans le deuxième état.

12. Aiguille à piquer selon l'une des revendications précédentes, dans laquelle l'aiguille à piquer (1) présente une pointe fermée (3), dans le premier état, et présente une ouverture (6) au niveau de la pointe (3), dans le deuxième état.

13. Aiguille à piquer selon l'une des revendications précédentes, dans laquelle l'aiguille à piquer (1) comporte un tuyau (7) de préférence élastique en un matériau à mémoire de forme, dans l'espace intérieur duquel un faisceau de fibres (8) tenu de manière étanche au moyen du tuyau (7) est disposé dans le premier état.

14. Aiguille à piquer selon l'une des revendications précédentes, dans laquelle l'aiguille à piquer (1) comprend un tuyau (7) qui présente dans sa paroi (5) et par tronçons des zones (10) en matériau à mémoire de forme.

15. Aiguille à piquer selon l'une des revendications précédentes, dans laquelle l'aiguille à piquer (1) comprend un tuyau (7) et des zones plissées (10) dont les couches plissées sont superposées, réalisés dans la paroi (5) du tuyau (7).

16. Aiguille à piquer selon l'une des revendications précédentes, dans laquelle l'aiguille à piquer (1) est formée au moins par tronçons avec un gel.

17. Aiguille à piquer selon la revendication précédente, dans laquelle l'aiguille à piquer (1) comprend un tuyau (7) qui comporte dans sa paroi (5) au moins par tronçons une ou plusieurs cavités (15) qui s'étendent en direction longitudinale de l'aiguille à piquer et qui sont remplies avec le gel.

18. Aiguille à piquer selon l'une des revendications précédentes, dans laquelle une modification d'état du premier état au deuxième état a lieu au moyen d'un agent de modification d'état qui exerce une tension électrique et/ou un rayonnement sur l'aiguille à piquer.

19. Aiguille à piquer selon l'une des revendications précédentes, dans laquelle la modification d'état a lieu par action d'une réaction chimique, d'un changement de température ou d'un changement de la valeur du pH sur l'aiguille à piquer.

20. Aiguille à piquer selon l'une des revendications précédentes, dans laquelle la modification d'état est réversible.

21. Aiguille à piquer selon l'une des revendications précédentes, dans laquelle, dans le premier état, un auxiliaire de piqûre (12 ; 13) dépasse au niveau de la pointe (3) de l'aiguille à piquer dans la direction longitudinale de l'aiguille au-delà d'une extrémité de l'aiguille à piquer (1), et dans le deuxième état, l'extrémité de l'aiguille à piquer (1) est déplacée au-delà de l'auxiliaire de piqûre (12 ; 13) en direction longitudinale de l'aiguille.

22. Aiguille à piquer selon l'une des revendications précédentes, dans laquelle l'aiguille à piquer comprend un tuyau (7) dont la paroi (5) comprend des zones rigides et des zones souples (10 ; 11) radialement par rapport à la direction longitudinale de l'aiguille à piquer.

23. Aiguille à piquer selon la revendication précédente, dans laquelle les zones souples (10) comprennent un plissage de la paroi.

24. Aiguille à piquer selon l'une des deux revendications précédentes, dans laquelle les zones souples (11) comportent une perforation de la paroi.

25. Aiguille à piquer selon l'une des revendications précédentes, dans laquelle l'aiguille à piquer (1) comporte un tuyau élastique (7) dans le lumen (4) duquel est reçue une structure de soutien (17) qui est rigide dans une direction le long de l'axe longitudinal de l'aiguille à piquer et qui est mobile dans une direction radiale par rapport à l'axe longitudinal de l'aiguille à piquer.

26. Aiguille à piquer selon la revendication précédente, dans laquelle la structure de soutien est réalisée par un ressort à spirale (17) dont les spires viennent se superposer.

27. Aiguille à piquer selon l'une des revendications précédentes, dans laquelle l'aiguille à piquer (1) comprend un tuyau élastique (7) qui inclut le lumen (4) et au moins un espace intermédiaire (19) agencé dans la paroi (5), un matériau de remplissage (20) étant prévu dans l'espace intermédiaire (19), matériau qui est comprimé dans un premier état par un agent de compression (21) et qui est détendu dans un deuxième état par échappement de l'agent de compression.

28. Aiguille à piquer selon la revendication précédente, dans laquelle l'agent de compression est une bague (21) déplaçable le long de l'axe longitudinal de l'aiguille à piquer et agencée dans le tuyau élastique (7).

29. Aiguille à piquer selon l'une des deux revendications précédentes, dans laquelle le matériau de remplissage (20) est ou contient un matériau en fibres, un matériau en granulés ou en particules, un gel et/ou un verre.
